# EUROPÄISCHE PATENTSCHRIFT

(11) **EP 0 675 175 B1**
(45) Veröffentlichungstag und Bekanntmachung des Hinweises auf die Patenterteilung: **31.05.2000**
(21) Anmeldenummer: 95104111.0
(22) Anmeldetag: 21.03.1995
(51) Int. Cl.: C09C 1/00, C09C 3/06, C09C 3/12, C09C 3/04, C09D 7/12, C09D 11/00, C08K 9/02, C08K 9/06, A61K 7/00, C09C 3/08

(54) **Kohlenstoffhaltige Pigmente**
Carbon containing pigments
Pigments contenant du carbone

(30) Priorität: 30.03.1994 DE 4410990; 01.10.1994 DE 4435300
(43) Veröffentlichungstag der Anmeldung: 04.10.1995
(73) Patentinhaber: MERCK PATENT GmbH, 64293 Darmstadt (DE)
(72) Erfinder: Bernhard, Klaus, D-64823 Gross-Umstadt (DE); Vogt, Reiner, Dr., D-64289 Darmstadt (DE); Pfaff, Gerhard, Dr., D-64839 Münster (DE)

(56) Entgegenhaltungen:
- EP-A- 0 499 864
- EP-A- 0 571 836
- EP-A- 0 634 458
- WO-A-93/12182

## Beschreibung

Die vorliegende Erfindung betrifft kohlenstoffhaltige Pigmente mit verbesserter Abriebfestigkeit sowie deren Herstellungsverfahren und Verwendung.

Es ist bekannt, daß durch Einarbeiten von Kohlenstoff in Pigmente besondere Farbeffekte erzielt werden können. Kohlenstoffhaltige Pigmente finden daher sowohl zur Pigmentierung von Lacken, Pulverlacken, Farben, Druckfarben, Kunststoffen und dergleichen, als auch in kosmetischen Zubereitungen Verwendung. Die aus dem Stand der Technik bekannten Verfahren zur Herstellung dieser Pigmente sind jedoch teilweise mit erheblichen Nachteilen behaftet.

Der bislang einzige Weg zur Herstellung von Kombinationen aus Kohlenstoff-Metalloxidglimmerpigmenten besteht im Aufbringen von Kohlenstoff aus wäßriger Suspension unter Verwendung geeigneter oberflächenaktiver Hilfsmittel oder durch Pyrolyse organischer Verbindungen. In der DE-AS 11 65 182 ist ein aufwendiges Pyrolyse-Verfahren beschrieben, wobei der Kohlenstoff sich naturgemäß lediglich auf der Pigmentoberfläche abscheidet. Der Nachteil des aufwendigen Pyrolyseverfahrens ist in der DE-PS 25 57 796 nicht mehr vorhanden. Bei dem dort beschriebenen Verfahren wird zunächst eine Substanz mit einer Rußdispersion vermischt. Durch Zugabe einer Metallsalzlösung unter Hydrolysebedingungen wird so eine rußhaltige Metallhydroxidschicht auf das Substrat aufgefällt. Die auf diese Weise hergestellten Pigmente werden abgetrennt und bei 130-150 °C getrocknet. Die so hergestellten Pigmente sind jedoch für verschiedene Verwendungszwecke ungeeignet, da sie unzureichende Abriebfestigkeiten besitzen. Gerade zur Einarbeitung in kosmetische Zubereitungen ist diese Eigenschaft äußerst unerwünscht. Durch das Glühen der Pigmente bei Temperaturen von 700-900 °C unter Sauerstoffausschluß läßt sich die Abriebfestigkeit verbessern (DE-OS 41 04 846). In der DE-OS 41 25 134 werden kohlenstoffhaltige Verbindungen in Gegenwart von Metalloxidplättchen oder mit Metalloxiden beschichteten plättchenförmigen Substraten unter Bedingungen pyrolisiert, bei denen das Metall des Metalloxids reduziert wird.

Bei den aus dem Stand der Technik bekannten Verfahren ist die Einfällung des Kohlenstoffes nicht quantitativ, d.h. der Kohlenstoff liegt auf dem Pigment zum Teil agglomeniert vor, so daß die Pigmente kein gutes Deckvermögen aufweisen. Der nicht eingefällte Kohlenstoff muß durch Sedimentation entfernt werden, was zeit- und kostenintensiv ist.

Ein weiterer Nachteil ist das häufig zu beobachtende Ausbluten des Kohlenstoffes beim Suspendieren der Pigmente in organischen Lösungsmitteln zur Herstellung von Lacksystemen.

Desweiteren besitzen diese Pigmente einen braunstichigen Charakter und zeichnen sich durch einen starken Glanzverlust aus, verursacht durch Absorption- und Streuphänomene der grobteiligen aufgefällten Kohlenstoffagglomerate.

Es bestand daher ein Bedürfnis zur Herstellung von kohlenstoffhaltigen Pigmenten mit verbesserter Abriebfestigkeit und Blutungsstabilität und hohem Glanz ohne das Erfordernis eines großen technischen Aufwandes. Diese Aufgabe konnte durch die vorliegende Erfindung gelöst werden.

Überraschenderweise wurde nun gefunden, daß kohlenstoffhaltige Pigmente mit einer verbesserten Abriebfestigkeit und besonderen Farbeffekten erhalten werden, wenn man Substrate, die mit ein oder mehreren Metalloxiden und organischen Kolloiden beschichtet sind, bei Temperaturen > 700 °C unter Sauerstoffausschluß glüht. Durch die Zersetzung der organischen Kolloidpartikeln unter inerten Bedingungen bei Temperaturen oberhalb 700 °C lassen sich direkt feinstteilige Kohlenstoffpartikel in der Metalloxidschicht ohne Agglomeration in gewünschter Menge herstellen.

Gegenstand der Erfindung sind somit kohlenstoffhaltige Pigmente erhältlich durch Pyrolyse von Substraten, die mit ein oder mehreren Metalloxiden und kolloidalen organischen Partikeln und gegebenenfalls mit einer organofunktionellen Silanverbindung beschichtet sind, bei Temperaturen > 700 °C unter Sauerstoffausschluß.

Gegenstand der Erfindung ist auch ein Verfahren zur Herstellung der kohlenstoffhaltigen Pigmente, dadurch gekennzeichnet, daß man eine wäßrige Substratsuspension herstellt und gleichzeitig aber separat mindestens eine hydrolysierbare Metallsalzlösung und eine wäßrige organische Kolloidlösung zugibt, wobei der pH-Wert der Substratsuspension durch gleichzeitige Zugabe einer Base oder einer Säure in einem Bereich gehalten wird, der die Metallsalzhydrolyse bewirkt, und gegebenenfalls zu dem auf diese Weise beschichteten Substrat eine organofunktionelle Silanverbindung und/oder eine wäßrige organische Kolloidlösung zugibt, und das auf diese Weise beschichtete Substrat abtrennt, wäscht, trocknet und bei Temperaturen >700 °C unter Sauerstoffausschluß glüht.

Geeignete Basissubstrate für die Beschichtung sind einerseits opake und andererseits transparente nicht-plättchenförmige Substrate. Bevorzugte Substrate sind Schichtsilikate sowie mit Metalloxiden beschichtete plättchenförmige Materialien. Insbesondere geeignet sind Glimmer, Talkum, Kaolin, Wismutoxichlorid, Glas-, SiO₂- oder synthetische Keramikflakes, synthetische trägerfreie Plättchen oder andere vergleichbare Materialien. Daneben kommen auch Metallplättchen, wie z.B. Aluminiumplättchen oder plättchenförmige Metalloxide, wie z.B. plättchenförmiges Eisenoxid und Glimmerbeschichtungen mit farbigen oder farblosen Metalloxiden, allein oder in Mischung in einer einheitlichen Schicht oder in aufeinanderfolgenden Schichten. Diese als Perlglanzpigmente bekannten Pigmente sind z.B. aus den deutschen Patenten und Patentanmeldungen 14 67 468, 19 59 998, 20 09 566, 22 14 545, 22 15 191, 22 44 298, 23 13 331, 25 22 572, 31 37 808, 31 37 809, 31 51 343, 31 51 354, 31 51 355, 32 11 602 und 32 35 017 bekannt.

Die plättchenförmigen Substrate haben in der Regel eine Dicke zwischen 0,1 und 5 µm und insbesondere zwischen 0,2 und 4,5 µm. Die Ausdehnung in den beiden anderen Bereichen beträgt üblicherweise zwischen 1 und 250 µm, insbesondere zwischen 2 und 200 µm.

Zur Herstellung der erfindungsgemäßen kohlenstoffhaltigen Pigmente wird zunächst eine wäßrige Suspension des Substrats hergestellt. In diese Suspension kann gegebenenfalls eine Lösung mindestens eines Metallsalzes eingebracht werden. Anschließend erfolgt die gleichzeitige, aber separate Zugabe einer weiteren Metallsalzlösung und der organischen Kolloidlösung, wobei der pH-Wert des Reaktionsgemisches durch gleichzeitige Zugabe einer Säure oder Base in einem Bereich gehalten wird, der die Metallsalzhydrolyse bewirkt. Dabei wird das Metalloxid zusammen mit den Kolloidpartikeln auf die Substratoberfläche aufgefällt.

Durch Variation der Dicke der dotierten Metalloxidschicht können insbesondere bei mit Titandioxid beschichteten plättchenförmigen Substraten beliebige Interferenzfarben erster oder höherer Ordnung erreicht werden.

Zur Fällung der Metallsalze kann jede Säure bzw. Base eingesetzt werden. Die optimalen Konzentrationen und pH-Werte können durch Routineversuche ermittelt werden. Üblicherweise wird der einmal zur Fällung eingestellte pH-Wert während der gesamten Fällung beibehalten um einheitliche Pigmente zu erzielen.

Zweckmäßigerweise wird man die technisch leicht zugänglichen Basen, wie z.B. NaOH, KOH oder Ammoniak, als Säuren verdünnte Mineralsäuren verwenden. Da die Basen und Säuren nur der pH-Wertänderung dienen, ist ihre Natur unkritisch, so daß auch andere Säuren und Basen eingesetzt werden können.

Nach dem Abtrennen, Waschen und Trocknen der auf diese Weise beschichteten Substrate werden die Pigmente bei Temperaturen > 700 °C, vorzugsweise bei 800-850 °C unter Sauerstoffausschluß geglüht, wobei die Zersetzung der organischen Kolloidpartikel stattfindet. Die Glühtemperatur hängt in der Regel von der aufgefällten Schichtdicke ab; die Glühdauer kann von einigen Minuten bis zu mehreren Stunden, vorzugweise jedoch zwischen 20 und 120 Minuten betragen.

Als Metallsalze, aus denen die Hydroxide gefällt werden können, sind alle wasserlöslichen Salze verwendbar, die durch Basen oder Säuren hydrolysierbar sind. Im allgemeinen ist die alkalische Hydrolyse bevorzugt. Geeignete Metallsalze sind insbesondere die von Aluminium, Titan, Zirkonium, Eisen, Chrom, Nickel, Cobalt und/oder Zinn.

Die einzelnen Verfahrensparameter zur Beschichtung bzw. zur Metallsalzhydrolyse sind bekömmlicher Art und ausführlich z.B. in der DE 25 57 796 beschrieben. Alle weiteren Parameter, wie z.B. Teilchengröße, Metallsalzkonzentrationen, Temperaturen und bevorzugte Ausführungsformen können ebenfalls der DE 25 57 796 entnommen werden.

Falls erwünscht können die erfindungsgemäßen Pigmente auch nachbeschichtet werden.

Wesentlicher Bestandteil des Belegungsmittels sind die organischen Kolloidpartikel. Alle bekannten organischen Kolloide mit Teilchenabmessungen < 10⁻⁵ cm können verwendet werden. Vorzugsweise werden gut wasserlösliche kolloidale organische Partikel, wie z.B. Polysaccharide, Stärke, Cellulose oder Gelatine sowie deren Derivate eingesetzt. Der Anteil an Kolloidpartikeln auf der Substratoberfläche beträgt 0,1 bis 20 Gew.%, vorzugsweise 0,5 bis 10 Gew.%, bezogen auf das Gesamtpigment.

Bei der Pyrolyse unter inerten Bedingungen zersetzt sich das Kolloid in der Metalloxidschicht, wobei ein äußerst feinteiliger Kohlenstoff mit Abmessungen < 5 nm erhalten wird. Der Kohlenstoff liegt gleichmäßig verteilt in der Metalloxidschicht vor. Zwischenräume zwischen den Metalloxiden werden aufgefüllt, so daß eine äußerst kompakte Kohlenstoff-Metalloxidschicht mit einem hohen Deckvermögen erhalten wird.

Der Kohlenstoffgehalt der Metalloxidschicht ist steuerbar durch die Menge an kolloidalen Partikeln, die auf der Substratoberfläche zusammen mit dem Metalloxid aufgebracht werden.

Der Anteil an Kohlenstoff in den erfindungsgemäßen Pigmenten liegt im allgemeinen zwischen 0,05 und 20 Gew.%, vorzugsweise zwischen 0,5 und 10 Gew.% und insbesondere zwischen 0,1 und 5 Gew.%, bezogen auf das Gesamtpigment.

Aufgrund der unterschiedlichen Substrate kann der gewichtsmäße Anteil an Kohlenstoff sehr stark variieren. Mit steigendem Kohlenstoffgehalt nimmt das Pigment einen immer graphitähnlicheren Glanz an.

Ein Ausbluten des Kohlenstoffs durch organische Lösungsmittel ist nicht zu beobachten, was auf die geringe Kohlenstoffpartikelgröße zurückzuführen ist. Weiterhin zeichnen sich die erfindungsgemäßen Pigmente durch einen gesteigerten Glanz als auch durch ihr hohes Deckvermögen aus. Die Pigmente sind ferner witterungsstabil und nicht leitfähig.

Zur Verbesserung der Photostabilität erfindungsgemäßer Pigmente, die eine Titandioxidschicht aufweisen, empfiehlt es sich während der Titansalzhydrolyse und der Kolloid-Fällung eine wasserlösliche Cer(III)-Verbindung mitzufällen.

Das Cer(III)salz kann in fester Form oder in wäßriger Lösung der Titansalzlösung zugesetzt werden oder gleichzeitig aber separat mit der Titansalzlösung und der Kolloidlösung zu der Substratsuspension zudosiert werden. Als Cer(III)-Verbindungen eignen sich insbesondere Cer(III)salze, vorzugsweise die Halogenide, insbesondere die Chloride. So hat sich bei den erfindungsgemäßen TiO₂-Pigmenten ein Cer(III)-Gehalt von 0,1-20 Gew.%, vorzugsweise 0,1-10 Gew.%, insbesondere 0,1-5 Gew.%, bezogen auf das Gesamtpigment als zweckmäßig erwiesen.

In Folge fehlender Oberflächenladung sind die so hergestellten Pigmente oft nur unzureichend suspendierbar. Die Dispergierbarkeit kann durch Nachbeschichtung mit einem Organosilan drastisch erhöht werden. Die Silanbeschichtung erfolgt unmittelbar an die Ce₂O₃/TiO₂-/ Kolloidbeschichtung. Geeignete Silanverbindungen sind organofunktionelle Silane der Formel

Y-(CH₂)ₙ-SiX₃,

worin
- Y: 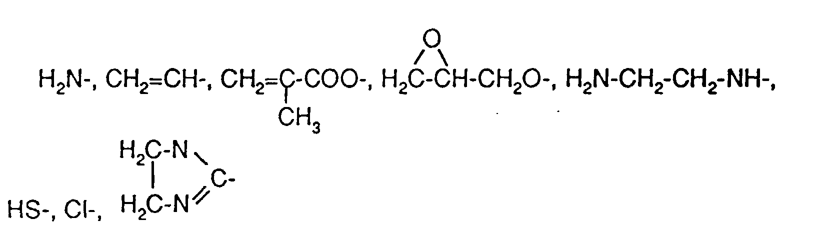
- X: OCH₃, OC₂H₅, OR, Cl, -OCOCH₃
- R: Alkyl mit 1-6 C-Atomen
- n: 0-3
bedeuten.

Vorzugsweise kommen Aminosilane zum Einsatz. Der Anteil der Silane beträgt 0,5-10 Gew.%, vorzugsweise 1-5 Gew.% und insbesondere 1-3 Gew.% bezogen auf das Gesamtpigment.

Anstelle der organofunktionellen Silanverbindungen kann man auch die bereits erwähnten kolloidalen Partikel in Mengen von 0,1 bis 10 Gew.%, vorzugsweise 0,5 bis 5 Gew.% verwenden. Weiterhin können auch Gemische aus organofunktionellen Silanverbindungen und kolloidalen Partikel in jedem Mischungsverhältnis eingesetzt werden. Vorzugsweise werden Aminosilane, Gelatine, Cellulose und Stärke verwendet.

Diese Nachbeschichtung, die unmittelbar an die Metallsalzhydrolyse und Kolloidfällung erfolgt, führt dazu, daß sich nach dem Glühen in einer Inertgasatmosphäre eine Kohlenstoffschicht auf der Pigmentoberfläche ausbildet, während in der Titandioxidschicht eingeschlossene Kohlenstoffpartikel vorliegen.

Die so hergestellten TiO₂-Pigmente, die im Eintopfverfahren hergestellt werden, zeigen nach dem Glühen augrund des Cer(III)-Gehaltes eine erhöhte Photostabilität und eine sehr gute Dispergierbarkeit, insbesondere in wäßrigen Systemen, durch die Silan- und/oder Kolloidnachbeschichtung.

Die erfindungsgemäß hergestellten Pigmente sind abriebfest, so daß sie für verschiedene Zwecke, insbesondere für Autolacke, für Druckfarben sowie in der Kosmetik eingesetzt werden können.

Gegenstand der Erfindung ist somit auch die Verwendung der kohlenstoffhaltigen Pigmente in Formulierungen wie Farben, Lacke, Druckfarben, Kunststoffen und Kosmetika.

Gegenstand der Erfindung sind weiterhin Formulierungen, die die erfindungsgemäßen Pigmente enthalten.

Die nachfolgenden Beispiele sollen die Erfindung näher verdeutlichen, ohne sie jedoch zu begrenzen:

### Beispiel 1 1 % SnO₂ + 4 % Gelatine + 38,6 % TiO₂ auf Glimmer

100 g Glimmer der Teilchengröße 10-60 µm werden in 2 l Wasser suspendiert und auf 75 °C erhitzt. Zu der Glimmersuspension wird eine Lösung bestehend aus 2,3 g SnCl₄ × 5 H₂O, 50 ml Wasser und 10 ml 5%ige HCl hinzudosiert. Der pH-Wert wird während der Zugabe mit 32%iger NaOH-Lösung konstant gehalten. Nach beendeter Zugabe der SnCl₄-Lösung wird zur Vervollständigung der Fällungsreaktion noch 0,5 h bei 75 °C und einem pH-Wert von 1,8 nachgerührt. Anschließend werden 250 ml TiCl₄-Lösung (368 g TiCl₄/l Wasser) und die Gelatine-Lösung / 4 g gelöst in 170 ml Wasser) gleichzeitig aber separat zu der Pigmentsuspension zudosiert.

Der pH-Wert wird während der Zugabe mit 32%iger NaOH-Lösung konstant gehalten. Nach Erreichen des gewünschten Silberfarbtons wird die Zugabe der TiCl₄-Lösung (∼ 250 ml) beendet. Anschließend wird 15 min bei 75 °C nachgerührt. Mit 32%iger NaOH-Lösung wird der pH-Wert auf 6,5 eingestellt und die Pigmentsuspension wird ohne Wärmebehandlung abermals 15 min gerührt. Man läßt die Pigmentsuspension absitzen und nach 1,5 h wird die überstehende Lösung abgesaugt. Zuletzt wird das Pigment salzfrei gewaschen, 18 h bei 140 °C getrocknet und in einer Stickstoffatmosphäre 45 min bei 850 °C geglüht.

### Beispiel 2

Analog Beispiel 1 erfolgt die Belegung von 100 g Glimmer mit SnO₂, TiO₂ und 4 g Dextrin (Stärkegummi der Fa. Merck, Darmstadt).

### Beispiel 3

Gemäß Beispiel 1 werden 100 g Glimmer der Teilchengröße 10-60 µm mit SnO₂, TiO₂ und 4 g Mowiol (Polyvinylalkohol der Fa. Hoechst, Frankfurt) belegt.

### Beispiel 4 SnO₂ + Ce₂O₃ + Gelatine + TiO₂ + Silan auf Glimmer

100 g Glimmer der Teilchengröße 10-60 µm werden in 2 l Wasser suspendiert und auf 75 °C erhitzt. Zu der Glimmersuspension wird eine Lösung bestehend aus 2,3 g SnCl₄ × 5 H₂O, 50 ml Wasser und 10 ml 37%ige HCl hinzudosiert. Der pH-Wert wird während der Zugabe mit 32%iger NaOH-Lösung konstant auf 1,8 gehalten. Nach beendeter Zugabe der SnCl₄-Lösung wird zur Vervollständigung der Fällungsreaktion noch 0,5 h bei 75 °C und einem pH-Wert von 1,8 nachgerührt. Anschließend wird eine Lösung bestehend aus 1,14g CeCl₃ · 7 H₂O und 20 ml Wasser unter Rühren zugegeben. Danach werden 250 ml TiCl₄-Lösung (368 g TiCl₄/l Wasser) und die Gelatine-Lösung / 4 g gelöst in 170 ml Wasser) gleichzeitig aber separat zu der Pigmentsuspension zudosiert. Der pH-Wert wird während der Zugabe mit 32%iger NaOH-Lösung konstant gehalten. Nach Erreichen des gewünschten Silberfarbtons wird die Zugabe der TiCl₄-Lösung (∼ 250 ml) beendet. Anschließend wird 15 min bei 75 °C nachgerührt. Mit 32%iger NaOH-Lösung wird der pH-Wert auf 6,0 eingestellt und die Pigmentsuspension wird ohne Wärmebehandlung abermals 5 min gerührt. Anschließend wird eine 2%ige wäßrige Silan-Lösung (2 g Z6040 der Fa. Hüls AG, Marl in 100 ml H₂O) innerhalb einer Minute zugegeben und 15 Minuten gerührt. Man läßt die Pigmentsuspension absitzen und nach 1,5 h wird die überstehende Lösung abgesaugt. Zuletzt wird das Pigment salzfrei gewaschen, 18 h bei 140 °C getrocknet und in einer Stickstoffatmosphäre 45 min bei 850 °C geglüht.

### Beispiel 5

Analog Beispiel 4 erfolgt die Belegung von 100 g Glimmer mit SnO₂, Ce₂O₃, TiO₂ und 4 g Gelatine. Zuletzt wird eine 0,5%ige Gelatinelösung (0,5 g Gelatine gelöst in 100 ml Wasser) innerhalb einer Minute zu der Pigmentsuspension zugegeben und 15 Minuten gerührt. Die Aufarbeitung erfolgt analog Beispiel 4.

### Beispiel 6

Gemäß Beispiel 4 werden 100 g Glimmer der Teilchengröße 10-60 µm mit SnO₂, Ce₂O₃, TiO₂, Mowiol (Polyvinylalkohol der Fa. Hoechst, Frankfurt) und 2 % Silan belegt.

### Beispiel 7

Gemäß Beispiel 4 werden 100 g Glimmer der Teilchengröße 10-60 µm mit SnO₂, C₂O₃, TiO₂, Mowiol (Polyvinylalkohol der Fa. Hoechst, Frankfurt) belegt. Anstelle der 2%igen Silan-Lösung wird eine 0,5%ige Mowiol-Lösung für die Nachbeschichtung verwendet. Die Aufarbeitung erfolgt analog Beispiel 4.

### Beispiel 8

Gemäß Beispiel 4 werden 100 g Glimmer der Teilchengröße 10-60 µm mit SnO₂, Ce₂O₃, TiO₂, Dextrin (Stärkegummi der Firma E. Merck, Darmstadt) und 2 % Silan belegt.

### Beispiel 9

Gemäß Beispiel 4 werden 100 g Glimmer der Teilchengröße 10-60 µm mit SnO₂, Ce₂O₃, TiO₂, Dextrin (Stärkegummi der Fa. E. Merck, Darmstadt) belegt. Anschließend wird eine 0,5%ige wäßrige Dextrin-Lösung (5 g Dextrin in 100 ml Wasser) innerhalb einer Minute zugegeben und 15 Minuten gerührt. Die Aufarbeitung erfolgt wie in Beispiel 4 beschrieben.

## Patentansprüche

1. Kohlenstoffhaltige Pigmente erhältlich durch Pyrolyse von Substraten, die mit ein oder mehreren Metalloxiden und kolloidalen organischen Partikeln und gegebenenfalls mit einer organofunktionellen Silanverbindung beschichtet sind, bei Temperaturen > 700 °C unter Sauerstoffausschluß.

2. Kohlenstoffhaltige Pigmente nach Anspruch 1, dadurch gekennzeichnet, daß die Substrate plättchenförmig sind.

3. Kohlenstoffhaltige Pigmente nach Anspruch 2, dadurch gekennzeichnet, daß die plättchenförmigen Substrate Glimmerplättchen, Glas-, SiO₂- oder synthetische Keramikflakes oder mit ein oder mehreren Metalloxiden beschichtete Glimmerplättchen sind.

4. Kohlenstoffhaltige Pigmente nach einem der Ansprüche 1 bis 3, dadurch gekennzeichnet, daß die kolloidalen organischen Partikel Stärke, Cellulose, Gelatine oder deren Derivate sind.

5. Kohlenstoffhaltige Pigmente nach Anspruch 1, dadurch gekennzeichnet, daß die organofunktionelle Silanverbindung eine Verbindung der Formel
Y-(CH₂)ₙ-SiX₃,
worin
Y 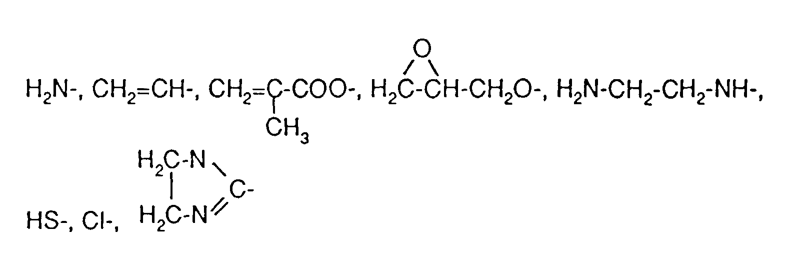
X OCH₃, OC₂H₅, OR, Cl, -OCOCH₃
R Alkyl mit 1-6 C-Atomen
n 0-3
bedeuten, ist.

6. Verfahren zur Herstellung kohlenstoffhaltiger Pigmente nach Anspruch 1, dadurch gekennzeichnet, daß man eine wäßrige Substratsuspension herstellt und gleichzeitig aber separat mindestens eine hydrolysierbare Metallsalzlösung und eine wäßrige organische Kolloidlösung zugibt, wobei der pH-Wert der Substratsuspension durch gleichzeitige Zugabe einer Base oder einer Säure in einem Bereich gehalten wird, der die Metallsalzhydrolyse bewirkt, und gegebenenfalls zu dem auf diese Weise beschichteten Substrat eine organofunktionelle Silanverbindung und/oder eine wäßrige organische Kolloidlösung zugibt, das fertige Substrat abtrennt, wäscht, trocknet und bei Temperaturen > 700 °C unter Sauerstoffausschluß glüht.

7. Verfahren nach Anspruch 6, dadurch gekennzeichnet, daß das Metallsalz ein Titansalz und eine Cer(III)salz ist.

8. Verwendung der kohlenstoffhaltigen Pigmente in Formulierungen wie Farben, Lacke, Druckfarben, Kunststoffen und Kosmetika.

9. Formulierungen enthaltend kohlenstoffhaltige Pigmente nach Anspruch 1.

## Claims

1. Carbon-containing pigments obtainable by pyrolysis of substrates coated with one or more metal oxides and colloidal organic particles and optionally with an organofunctional silane compound at temperatures > 700°C under oxygen-excluding conditions.

2. Carbon-containing pigments according to Claim 1, characterized in that the substrates are plateletlike.

3. Carbon-containing pigments according to Claim 2, characterized in that the plateletlike substrates are mica platelets, or glass, SiO₂ or synthetic ceramic flakes or mica platelets coated with one or more metal oxides.

4. Carbon-containing pigments according to any one of Claims 1 to 3, characterized in that the colloidal organic particles are starch, cellulose, gelatin or derivatives thereof.

5. Carbon-containing pigments according to Claim 1, characterized in that the organofunctional silane compound is a compound of the formula
Y-(CH₂)ₙ-SiX₃
where
Y is 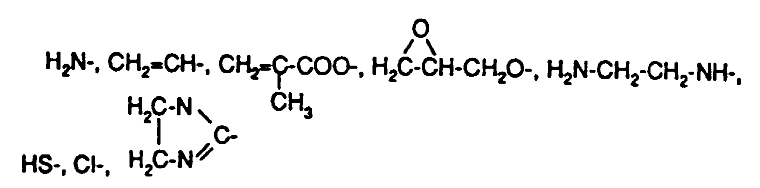
X is OCH₃, OC₂H₅, OR, Cl, -OCOCH₃
R is alkyl having 1-6 carbon atoms
n is 0-3.

6. Process for producing carbon-containing pigments according to Claim 1, characterized in that an aqueous substrate suspension is prepared and admixed simultaneously but separately with at least one hydrolysable metal salt solution and with an aqueous organic colloid solution, the pH of the substrate suspension being maintained by simultaneous addition of a base or an acid within a range which causes the metal salt to hydrolyse, and the substrate coated in this way optionally has added to it an organo-functional silane compound and/or an aqueous organic colloid solution, and the ready-produced substrate is separated off, washed, dried and calcined at temperatures > 700°C under oxygen-excluding conditions.

7. Process according to Claim 6, characterized in that the metal salt is a titanium salt and a cerium(III) salt.

8. Use of the carbon-containing pigments in formulations such as paints, coatings, printing inks, plastics and cosmetics.

9. Formulations comprising carbon-containing pigments according to Claim 1.

## Revendications

1. Pigments carbonés pouvant être obtenus par pyrolyse de substrats, qui sont enrobés avec un ou plusieurs oxydes métalliques et des particules organiques colloïdales et éventuellement avec un silane organofonctionnel, à des températures > 700°C et à l'abri de l'oxygène.

2. Pigments carbonés selon la revendication 1, caractérisés en ce que les substrats sont en paillettes.

3. Pigments carbonés selon la revendication 2, caractérisés en ce que les substrats en paillettes sont des paillettes de mica, des paillettes de verre, SiO₂ ou céramique synthétique, ou des paillettes de mica enrobées d'un ou plusieurs oxydes métalliques.

4. Pigments carbonés selon l'une des revendications 1 à 3, caractérisés en ce que les particules organiques colloïdales sont des particules d'amidon, de cellulose, de gélatine ou de leurs dérivés.

5. Pigments carbonés selon la revendication 1,
caractérisés en ce que le silane organofonctionnel est un composé de formule
Y-(CH₂)ₙ-SiX₃
dans laquelle
Y représente 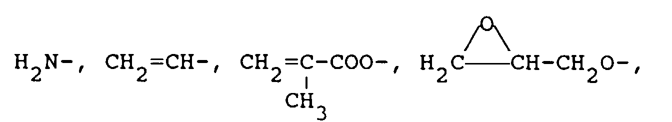 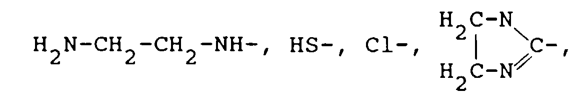
X représente OCH₃, OC₂H₅, OR, Cl, -OCOCH₃,
R représente un groupe alkyle ayant de 1 à 6 atomes de carbone,
n va de 0 à 3.

6. Procédé pour la préparation de pigments carbonés selon la revendication 1, caractérisé en ce que l'on prépare une suspension aqueuse de substrat et on y ajoute, simultanément mais séparément, au moins une solution hydrolysable de sel métallique et une solution aqueuse organique colloïdale, le pH du substrat étant maintenu, par addition simultanée d'une base ou d'un acide, dans un intervalle qui provoque l'hydrolyse du sel métallique, et éventuellement on ajoute au substrat enrobé de cette façon un silane organofonctionnel et/ou une solution aqueuse organique colloïdale, le substrat final est séparé, lavé, séché et calciné à des températures > 700°C, à l'abri de l'oxygène.

7. Procédé selon la revendication 6, caractérisé en ce que le sel métallique est un sel de titane et un sel de cérium-(III).

8. Utilisation des pigments carbonés dans des formulations telles que des couleurs, des vernis, des encres d'imprimerie, des matières plastiques et des cosmétiques.

9. Formulations contenant des pigments carbonés selon la revendication 1.
